# EUROPEAN PATENT APPLICATION

(11) **EP 3 878 458 A1**
(43) Date of publication of application: **15.09.2021**
(21) Application number: 19882207.4
(22) Date of filing: 06.11.2019
(51) Int. Cl.: A61K 35/28, A61K 35/545, A61K 31/215, A61P 37/06, C12N 5/0775

(54) **PHARMACEUTICAL COMPOSITION CONTAINING PROTEIN KINASE C ACTIVATOR-TREATED STEM CELLS OR CULTURE THEREOF FOR PREVENTING OR TREATING AUTOIMMUNE DISEASES**

(30) Priority: 09.11.2018 KR 20180137578
(71) Applicant: Corestem Co., Ltd., Cheongju-si, Chungcheongbuk-do 28420 (KR)
(72) Inventor: KIM, Kyung Suk, Seoul 02838 (KR); LEE, Tae Yong, Yongin-si, Gyeonggi-do 17128 (KR)
(74) Representative: J A Kemp LLP
(86) International application number: PCT/KR2019/014960
(87) International publication number: WO 2020/096340

(57) **Abstract**

The present invention relates to a pharmaceutical composition for preventing or treating an autoimmune disease, including stem cells treated with a protein kinase C activator or a culture thereof, and more specifically, the present invention relates to a pharmaceutical composition for preventing or treating an autoimmune disease, which can inhibit the function of B cells and have an excellent prophylactic or therapeutic effect for an autoimmune disease.

## Description

### CROSS-REFERENCE TO RELATED APPLICATION

This application claims priority to and the benefit of Korean Patent Application No. 10-2018-0137578, filed on November 9, 2018, the disclosure of which is incorporated herein by reference in its entirety.

### [Technical Field]

The present invention relates to a pharmaceutical composition for preventing or treating an autoimmune disease, including stem cells treated with a protein kinase C activator or a culture thereof, and more specifically, the present invention relates to a pharmaceutical composition for preventing or treating an autoimmune disease, which can inhibit the function of B cells and have an excellent prophylactic or therapeutic effect for an autoimmune disease.

### [Background Art]

Stem cells are collectively referred to as undifferentiated cells at a stage before being differentiated into each cell constituting tissues, and differentiation into specific cells proceeds by specific differentiation stimuli (environment). Unlike differentiated cells in which cell division is stopped, stem cells can produce cells which are identical to themselves (self-renewal) by cell division, and thus, they have the property of proliferation (expansion). In addition, when a differentiation stimulus is applied, they differentiate into specific cells, and since they can be differentiated into other cells by different environments or different differentiation stimuli, they are characterized in having plasticity of differentiation.

The pluripotency of these stem cells provides a good *in vitro* experimental model for the study of human developmental processes. New drug development can be facilitated by performing drug tests and toxicity tests on homogeneous human tissues or cells obtained from stem cells. Furthermore, it is possible to obtain a large amount of cells or tissues that can replace damaged tissues, which can be used to treat incurable diseases.

Mesenchymal stem cells are cells that maintain sternness and self-renewal and have the capacity to differentiate into various mesenchymal tissues (plasticity). These cells can be extracted from bone marrow, adipose tissue, umbilical cord blood, synovial membrane, trabecular bone, infrapatellar fat pad, and the like. Mesenchymal stem cells inhibit the activity and proliferation of T lymphocytes and B lymphocytes, suppress the activity of natural killer cells (NK cells), and have immunomodulatory capacity to regulate the functions of dendritic cells and macrophages, and thus, these are cells capable of allotransplantation and xenotransplantation. In addition, mesenchymal stem cells have the ability to differentiate into various connective tissues such as cartilage, bone tissue, ligaments, bone marrow matrix, and the like. In particular, adipose-derived mesenchymal stem cells derived from adipose tissue are subdivided into names such as adipose-derived stem/stromal cells (ASC), adipose-derived adult stem cells (ADAS), or the like, and these can be applied to the production of biomaterials to treat soft tissue defects caused by trauma, tumor removal surgery, burns, and the like.

Meanwhile, lupus activates dendritic cells, T cells, and B cells by autoantigens as an abnormal immune response. Therefore, autoantibodies are produced, and symptoms appear by invading various organs by forming immune complexes with autoantigens. Various therapeutic agents have been developed to treat such lupus disease, and in particular, mesenchymal stem cells (MSC) having immunomodulatory action are being studied as promising therapeutic agents. Although MSC has been reported to inhibit the function of T cells, studies on MSC capable of inhibiting the function of B cells are insufficient.

### Related Art Documents

Korean Patent Laid-Open No. 10-2015-0144679

### [Disclosure]

### [Technical Problem]

The present invention provides a pharmaceutical composition for preventing or treating an autoimmune disease, including stem cells treated with a protein kinase C activator or a culture thereof.

In addition, the present invention provides a method for preventing or treating an autoimmune disease, including administering the pharmaceutical composition to a subject other than humans.

In addition, the present invention provides a method for preparing an immunosuppressive agent, including culturing by adding a protein kinase C activator to stem cells.

In addition, the present invention provides a method for inhibiting an immune response of a subject other than humans, including administering stem cells treated with a protein kinase C activator or a culture thereof to the subject.

However, the technical problems to be achieved by the present invention are not limited to the problems mentioned above, and other problems that are not mentioned will be clearly understood by those skilled in the art from the following description.

### [Technical Solution]

The present invention provides a pharmaceutical composition for preventing or treating an autoimmune disease, including stem cells treated with a protein kinase C activator or a culture thereof.

The protein kinase C activator may include one or more selected from the group consisting of phorbol myristate acetate, ingenol 3-angelate, bryostatin-1, phorbol-12,13-dibutyrate, prostatin, N-(6-phenylhexyl)-5-chloro-1-naphthalenesulfonamide (SC-9), and 5-chloro-N-heptylnaphthalene-1-sulfonamide (SC-10).

The stem cells may be adult stem cells, pluripotent stem cells, induced pluripotent stem cells, or embryonic stem cells.

The adult stem cells may be mesenchymal stem cells, mesenchymal stromal cells, or multipotent stem cells.

The protein kinase C activator may increase the expression of CXCL10 in stem cells.

The protein kinase C activator may increase apoptosis of B cells by increasing the expression of programmed death-ligand 1 (PD-L1) in stem cells.

The autoimmune disease may be selected from the group consisting of lupus (systemic lupus erythematosus), rheumatoid arthritis, progressive systemic sclerosis (scleroderma), atopic dermatitis, alopecia areata, psoriasis, pemphigus, asthma, aphthous stomatitis, chronic thyroiditis, inflammatory enteritis, Behcet's disease, Crohn's disease, dermatomyositis, polymyositis, multiple sclerosis, autoimmune hemolytic anemia, autoimmune encephalomyelitis, myasthenia gravis, Grave's disease, polyarteritis nodosa, ankylosing spondylitis, fibromyalgia syndrome, and temporal arteritis.

In addition, the present invention provides a method for preventing or treating an autoimmune disease, including administering the pharmaceutical composition to a subject other than humans.

In addition, the present invention provides a method for preparing an immunosuppressive agent, including culturing by adding a protein kinase C activator to stem cells.

In addition, the present invention provides a method for inhibiting an immune response of a subject other than humans, including administering stem cells treated with a protein kinase C activator or a culture thereof to the subject.

In addition, the present invention provides a method for preventing or treating an autoimmune disease, including administering or feeding a pharmaceutical composition including stem cells treated with a protein kinase C activator or a culture thereof to a subject.

In addition, the present invention provides a use of a pharmaceutical composition including stem cells treated with a protein kinase C activator or a culture thereof in the prevention or treatment of an autoimmune disease.

### [Advantageous Effects]

The stem cells or a culture thereof according to the present invention may be effectively used for the prevention or treatment of autoimmune diseases. More specifically, since stem cells treated with a protein kinase C activator or a culture thereof can inhibit the function of B cells, they have an excellent prophylactic or therapeutic effect for autoimmune diseases.

### [Description of Drawings]

FIG. 1 is an analysis of the effect of PMA-treated MSC on B cell function. * = p < 0.05, ** = p < 0.01, *** = p < 0.001 [one-way ANOVA, Tukey's test]
FIG. 2 is an analysis of the effect of PMA-treated MSC on B cell migration. * = p < 0.05, ** = p < 0.01, *** = p < 0.001 [one-way ANOVA, Tukey's test]
FIG. 3 is an analysis of the effect of PMA-treated MSC on B cell contact. * = p < 0.05, ** = p < 0.01, *** = p < 0.001 [one-way ANOVA, Tukey's test]
FIG. 4 is an analysis of the effect of PDL1 expressed in PMA-treated MSC on B cell function. * = p < 0.05, ** = p < 0.01, *** = p < 0.001 [one-way ANOVA, Tukey's test]
FIG. 5 is an analysis of the effect of PDL1 expressed in PMA-treated MSC on B cell apoptosis. * = p < 0.05, ** = p < 0.01, *** = p < 0.001 [one-way ANOVA, Tukey's test]
FIG. 6 confirms the therapeutic effect of MRL/lpr mice by PMA-treated hMSC administration. * = p < 0.05, ** = p < 0.01, *** = p < 0.001 [one-way ANOVA, Tukey's test]
FIG. 7 is an analysis of immune cells in the kidney of MRL/lpr mice by PMA-treated hMSC administration. * = p < 0.05, ** = p < 0.01, *** = p < 0.001 [one-way ANOVA, Tukey's test]

### [Modes of the Invention]

Hereinafter, the present invention will be described in more detail.

The present invention provides a pharmaceutical composition for preventing or treating an autoimmune disease, including stem cells treated with a protein kinase C activator or a culture thereof.

As used herein, the term "stem cells" may be cells having the ability to differentiate into various tissues, that is, 'undifferentiated cells.'

In the present invention, the stem cells may be adult stem cells, pluripotent stem cells, induced pluripotent stem cells, or embryonic stem cells.

The adult stem cells may be derived from tissue selected from the group consisting of umbilical cord, umbilical cord blood, bone marrow, fat, muscle, nerve, skin, amniotic membrane, and placenta. In addition, the adult stem cells may be mesenchymal stem cells, mesenchymal stromal cells, or multipotent stem cells, and preferably, mesenchymal stem cells. The method for obtaining stem cells from each tissue may be by a method known in the art, and it is not limited to the methods of the exemplary embodiments of the present invention.

As used herein, the term "culture" may include a cell culture solution including stem cells, a culture supernatant from which stem cells are removed from a cell culture solution, and a diluted solution thereof. The composition of the culture may additionally include components required for conventional stem cell culture, as well as components that act synergistically on stem cell proliferation, and accordingly, the composition may be easily selected by a person having ordinary skill in the art.

As the medium used for culturing the stem cells, a conventional medium known to be suitable for stem cell culture in the art may be used, for example, Dulbecco's modified Eagle medium (DMEM) or keratinocyte serum free medium (keratinocyte-SFM) may be used.

The stem cell medium may be supplemented with additives. In general, it may contain a neutral buffer (e.g., phosphate and/or high-concentration bicarbonate) and a protein nutrient (*e.g.,* serum such as FBS, serum substitutes, albumin, or essential amino acids and non-essential amino acids such as glutamine) in isotonic solutions. Furthermore, it may contain lipids (fatty acid, cholesterol, HDL or LDL extracts of serum) and other components found in most preservative media of this kind (*e.g.,* insulin or transferrin, nucleosides or nucleotides, pyruvate, sugars in any ionized form or salt such as glucose, selenium, glucocorticoids, for example, hydrocortisone and/or reducing agents, and for example, β-mercaptoethanol).

In the composition of the present invention, the protein kinase C activator may include one or more selected from the group consisting of phorbol myristate acetate (PMA), ingenol 3-angelate (I3A), bryostatin-1, phorbol-12,13-dibutyrate (PDBu), prostatin, SC-9 (N-(6-phenylhexyl)-5-chloro-1-naphthalenesulfonamide, CAS No. 102649-78-5), and SC-10 (5-chloro-N-heptylnaphthalene-1-sulfonamide, CAS No. 102649-79-6).

The treatment concentration of the protein kinase C activator may be 1 µg/mL to 20 µg/mL. The treatment time of the protein kinase C activator may be 1 hour to 120 hours.

The protein kinase C activator may increase the expression of CXCL10 in stem cells to increase B cell migration, thereby increasing the effect of contact-dependent inhibition. In addition, the protein kinase C activator may increase the expression of programmed death-ligand 1 (PD-L1) in stem cells to increase apoptosis of B cells.

According to the present invention, mesenchymal stem cells that are treated with a protein kinase C activator may inhibit the function of B cells, and the stem cells and a culture thereof according to the present invention may be effectively used in the prevention and treatment of autoimmune diseases.

The autoimmune disease may be selected from the group consisting of lupus (systemic lupus erythematosus), rheumatoid arthritis, progressive systemic sclerosis (scleroderma), atopic dermatitis, alopecia areata, psoriasis, pemphigus, asthma, aphthous stomatitis, chronic thyroiditis, inflammatory enteritis, Behcet's disease, Crohn's disease, dermatomyositis, polymyositis, multiple sclerosis, autoimmune hemolytic anemia, autoimmune encephalomyelitis, myasthenia gravis, Grave's disease, polyarteritis nodosa, ankylosing spondylitis, fibromyalgia syndrome, and temporal arteritis.

As used herein, the term "prevention" refers to all actions that suppress or delay the onset of autoimmune diseases through administration of the composition, and the term "treatment" refers to all actions that alleviate or ameliorate the symptoms of autoimmune diseases through administration of the composition.

In addition, the stem cells or a culture thereof according to the present invention may include 1.0 × 10⁵ to 1.0 × 10⁹ cells per 1 mL, preferably, 1.0 × 10⁶ to 1.0 × 10⁸ cells per 1 mL, and more preferably, 1.0 × 10⁷ cells per 1 mL.

The stem cells or a culture thereof according to the invention may be used without freezing or may be frozen for future use. If it is necessary to be frozen, standard cryopreservatives (*e.g.,* DMSO, glycerol, and the EpiLife cell freeze medium (Cascade Biologics)) may be added to a cell population prior to freezing.

In another aspect, the present invention provides a method for preventing or treating an autoimmune disease, including administering the pharmaceutical composition to a subject other than humans.

As used herein, the term "administration" means the introduction of a given substance into a subject by an appropriate method.

As used herein, the term "individual" means all animals, such as mice, rats, livestock, and the like, including humans that have or may develop an autoimmune disease. As a specific example, it may be a mammal, including a human.

In addition, the stem cells or a culture thereof according to the present invention may be formulated and administered into a preparation as a unit dosage form suitable for administration into a patient's body according to a conventional method in the pharmaceutical field, and the preparation includes an effective dose to be administered once or several times. Suitable formulations for this purpose as parenteral administration preparations may preferably be injections such as ampoules for injection, infusions such as infusion bags, sprays such as aerosol preparations, and the like. The ampoule for injection may be mixed with an injection solution immediately before use, and physiological saline, glucose, mannitol, Ringer's solution, and the like may be used as the injection solution. In addition, the infusion bag may be made of a polyvinyl chloride or polyethylene material, and infusion bags from Baxter, Becton Dickinson, Medcep, National Hospital Products, or Terumo may be exemplified.

The pharmaceutical preparation may further include one or more pharmaceutically acceptable conventional inert carriers other than the active ingredient, for example, preservatives, painless agents, solubilizers, stabilizers, or the like in the case of injections, and bases, excipients, lubricants, preservatives, or the like in the case of preparations for topical administration.

The stem cells according to the present invention, a culture thereof, or a pharmaceutical preparation thereof prepared as described above may be administered with other stem cells used for transplantation and other uses by using an administration method conventionally used in the art, or in the form of a mixture with such stem cells. Preferably, it is possible to directly engraft or implant into the diseased area of a patient in need of treatment, or directly implant or inject into the abdominal cavity, but is not limited thereto. In addition, the administration is possible both by non-surgical administration using a catheter or surgical administration methods such as infusion or implantation after incision of a disease site, but a non-surgical administration method using a catheter is more preferable. Also, in addition to administering parenterally according to a conventional method, for example, directly into the lesion, transplantation by intravascular injection, which is a general method of hematopoietic stem cell transplantation, is also possible.

The daily dose of the stem cells may be administered by dividing into 1.0 × 10⁴ to 1.0 × 10¹⁰ cells/kg of body weight, preferably, 1.0 × 10⁵ to 1.0 × 10⁹ cells/kg of body weight once or several times. However, it should be understood that the actual dosage of the active ingredient should be determined in light of various related factors such as the disease to be treated, the severity of the disease, the route of administration, the patient's weight, age and sex, and thus, the dosage does not limit the scope of the present invention in any way.

In another aspect, the stem cells treated with a protein kinase C activator and a culture thereof according to the present invention may be administered to suppress an autoimmune reaction, and the present invention provides a method for inhibiting an immune response in a subject, including administering stem cells treated with a protein kinase C activator or a culture thereof to the subject.

As used herein, the term "subject" refers to mammals including cows, dogs, pigs, chickens, sheep, horses, and humans, but is not limited thereto. Moreover, preferably, the administration of stem cells or a culture thereof to which a protein kinase C activator is added may be intraperitoneal or intravascular administration, direct administration into a lesion, administration into a synovial cavity of the joint, or the like.

The suppression of the immune response may be characterized by preventing or treating an immune disease.

In another aspect, the present invention provides a method for preparing an immunosuppressive agent, including culturing by adding a protein kinase C activator to stem cells.

As used herein, the term "immunosuppressive agent" as described above in the present invention refers to a preparation that can cure autoimmune diseases by suppressing an immune response, as a preparation including stem cells obtained by treating a protein kinase C activator to stem cells or a culture thereof.

Hereinafter, the present invention will be described in more detail through exemplary embodiments. The objects, features, and advantages of the present invention will be readily understood through the following exemplary embodiments. The present invention is not limited to the exemplary embodiments described herein, and may be embodied in other forms. The exemplary embodiments introduced herein are provided to sufficiently convey the spirit of the present invention to those skilled in the art to which the present invention pertains. Therefore, the present invention should not be limited by the following exemplary embodiments.

As an abnormal immune response, lupus activates dendritic cells, T cells, and B cells by autoantigens. Accordingly, autoantibodies are produced, and autoantigens and immune complexes are formed and invade various organs to show symptoms. Various therapeutic agents have been developed to treat such lupus disease, and mesenchymal stem cells (MSC), which have immunomodulatory effects, are being studied as promising therapeutic agents. It has been reported that MSC inhibits the function of T cells. However, it has not been clearly confirmed whether MSC inhibits the function of B cells. Accordingly, the present inventors confirmed whether human MSC inhibits the function of human B cells through the following exemplary embodiments.

The present inventors confirmed that human MSC does not inhibit the production of IgM in human B cells and slightly increases the same through preliminary experiments. The present inventors treated 20 types of chemicals to human MSC to prepare human MSC that inhibits human B cell function. Through screening, it was confirmed that phorbol myristate acetate (PMA), which is a protein kinase C activator, activates human MSC and ultimately inhibits human B cell function, and the mechanism of action of a protein kinase C activator activating human MSC was investigated.

### <Example>

### Materials and methods

### Materials

MRL/lpr mice were purchased from Jackson Laboratory (Bar Harbor, Maine, USA). The mice were stored at a specific pathogen-free state under a condition of a temperature at 21°C to 24°C and a relative humidity at 40% to 60% under a 12-hour light/dark cycle.

Human mesenchymal stem cells (MSC) were obtained from bone marrow (BM) cells of human tibiae and femurs. BM cells were cultured in α-MEM medium including 10% fetal bovine serum (FBS), 2 mM L-glutamine, and penicillin/streptomycin under a condition of 37°C and 5% CO₂. Non-adherent cells were removed on day 1, and adhered cells were cultured with medium replenishment every 3 days and used between day 17 and day 20.

By using a phenomenon in which gene expression is inhibited sequence-specifically by 12 to 21 mer dsRNA called small interfering RNA (siRNA) (manufactured by Bioneer, Korea), CXCL10, CXCL12, and CCL2-knockdown hMSCs were cultured for 48 hours to induce knockdown reactions.

As substances used for the pretreatment condition for hMSC, PMA (10 ng/mL, 24-hour treatment, Sigma), I3A (10 µg/mL, 24-hour treatment, Sigma), and IFN-γ (10 ng/mL, 7-day treatment, Sigma) were used.

### ELISA

IFN-γ measurement was performed according to the test method provided by R&D (#DY285-05), and for IgM, the IgM ELISA kit (eBioscience, Vienna, Austria) was used for measurement. The capture antibody and the coating buffer were diluted at a ratio of 1:250 in 96 wells for ELISA and coated at 4°C for 18 hours. After washing twice using a 200 µL of a wash solution, 250 µL of a blocking buffer was placed in a well, and then, it was reacted at room temperature for 2 hours. Afterwards, washing was performed twice using 200 µL of a wash solution, and the sample was prepared by diluting a specimen in an assay buffer (1X). In each well, 90 µL of an assay buffer (1x), 50 µL of a detection antibody, and 10 µL of a standard or the sample were added, and it was reacted at room temperature for 3 hours. After the reaction was completed, washing was performed using a washing solution. 100 µL of a substrate solution was added to each well, followed by reacting for 5 minutes, and 100 µL of a stop solution was added to stop the reaction. Finally, the value was measured at 450 nm to 570 nm.

### Western blot

Cells were lysed in ice using a cell lysis buffer (Cell Signaling Technology, Danvers, MA, USA). Proteins were obtained by centrifugation at 12,000 rpm for 15 minutes at 4°C. The proteins were quantified using the Bradford reagent, and electrophoresis was performed at 75V with 20 µg of the proteins by using an SDS-polyacrylamide gel. After the SDS-polyacrylamide gel was transferred to a PVDF membrane at 95V for 90 minutes, 5% nonfat dry milk was added to TBS (TTBS) including 0.5% Tween 20 and blocked for 1 hour. After blocking, the membrane was placed in 5% BSA/TTBS including a primary antibody and shaken at 4°C for one day. The next day, after washing the membrane with TTBS, the membrane was placed in 5% BSA/TTBS including a secondary antibody, and the secondary antibody was attached at room temperature for 90 minutes. After washing the membrane with TTBS and reacting the membrane with enhanced chemiluminescence (ECL, Amersham Pharmacia Biotech, Piscataway, NJ, USA), the degree of protein expression was measured using a ChemiDoc™ XRS+ machine (Bio-Rad, CA, USA).

### RNA isolation using the Trizol method

Cells were treated with Trypsin-EDTA (Gibco) at 0.125% at a time point when the confluency of 70% to 80% was reached, followed by culturing under a condition of 5% CO₂ and 37 °C. After 24 hours of material treatment, the cells were recovered, and the total RNA was isolated from the cells using the TRIZOL reagent (Invitrogen, MD, USA), and the total RNA was quantified by absorbance measurement at 260 nm.

### Reverse transcriptase-PCR

A total RNA concentration of 0.3 µg was used, and it was synthesized at 42°C for 1 hour and at 95°C for 5 minutes. Polymerase chain reaction was performed using 3 µL of synthesized cDNA and 10 pM of a primer. For the basic process of this reaction, the pre-denaturing phase was performed at 94°C for 5 minutes, the denaturing phase was performed at 94°C for 30 seconds, the annealing phase was performed at 56°C for 30 seconds, the elongation phase was performed at 72°C for 1 minute, and the post-elongation phase was performed at 72°C for 5 minutes. Electrophoresis was performed by making a 1% agarose gel.

### RT-PCR

The expression levels of mRNA for FasL, PD-L1, PD-L2, ICAM1, VCAM1, β-actin, IFN-γ, TNF-α, IL-12, COX-2, iNOS, IDO, TGF-β, CCL2, CCL3, CXCL10, and CXCL12 were analyzed by quantitative real-time PCR (qPCR). The relative mRNA amount of each sample was calculated based on a threshold cycle (Ct) compared to the threshold cycle (Ct) of β-actin, which is a housekeeping gene.

### Annexin V staining

After washing the cells twice with cold PBS, the cells were released in a binding buffer at a concentration of 1 × 10⁶ cells/mL. Then, after transferring an amount of 100 µL to a 5 mL culture tube, the cells were stained by passing through an incubation process for 15 minutes at 25°C room temperature, and under a dark condition using 5 µL of the Annexin V Apoptosis Detection Kit (BD Pharmingen, USA). Then, after adding 400 µL of a binding buffer solution, it was measured using flow cytometry (Canto II, BD Bioscience).

### Primer sequence information

The information of the used primers is as follows. CCL2 forward primer 5-'ATG AAA GTC TCT GCC GCC CTT CTG T-3', CCL2 reverse primer 5-'AGT CTT CGG AGT TTG GGT TTG CTT G-3', CCL3 forward primer 5'-ATG CAG GTC TCC ACT GCT GCC CTT-3 ', CCL3 reverse primer 5'-GCA CTC AGC TCC AGG TCG CTG ACA T-3', CXCL10 forward primer 5'-CCT GCT TCA AAT ATT TCC CT-3 ', CXCL10 reverse primer 5'-CCT TCC TGT ATG TGT TTG GA-3 ', CXCL12 forward primer 5'-ATG AAC GCC AAG GTC GTG GTC G-3', CXCL12 reverse primer 5'-TGT TGT TGT TCT TCA GCC G-3 ', COX-2 forward primer 5 '-TCC TTG CTG TTC CCA CCC AT-3', COX-2 reverse primer 5'-CAT CAT CAG ACC AGG CAC CA-3 ', iNOS forward primer 5'-ACG TGC GTT ACT CCA CCA AC-3', iNOS reverse primer 5'-CAT AGC GGA TGA GCT GAG CA-3 ', IDO forward primer 5'-AGCC TGA TCT CAT AGA GTC TG-3', IDO reverse primer 5'-TTA CTG CAG TCT CCA TCA CG-3 ', TGF-β forward primer 5'-CAG ATC CTG TCC AAG CTG-3 ', TGF-β reverse primer 5'-TCG GAG CTC TGA TGT GTT-3', FasL forward primer 5'-GGA TTG GGC CTG GGG ATG TTT CA -3 ', FasL reverse primer 5'-TTG TGG CTC AGG GGC AGG TTG TTG-3 ', PD-L1 forward primer 5'-TTG GGA AAT GGA GGA TAA GA-3', PD-L1 reverse primer 5'-GGA TGT GCC AGA GGT AGT TCT-3 ', PD-L2 forward primer 5'-ACA CCG TGA AAG AGC C-3 ', PD-L2 reverse primer 5'-AAT GTG AAG CAG CCA AG-3', ICAM1 forward primer 5'-CGT GCC GCA CTG AAC TGG AC-3 ', ICAM1 reverse primer 5'-CCT CAC ACT TCA CTG TCA CCT-3 ', VCAM1 forward primer 5'-ATG ACA TGC TTG AGC CAG G-3', VCAM1 reverse primer 5'-GTG TCT CCT TCT TTG ACA CT-3 ', β -actin forward primer 5'-GTG GGG CGC CCC AGG CAC CA-3 ', β-actin reverse primer 5'-CTC CTT AAT GTC ACG CAC GA-3'. The mouse primers used were INF-γ forward primer 5'-AGC GGC TGA CTG AAC TCA GAT TGT AG-3 ', INF-γ reverse primer 5'-GTC ACA GTT TTC AGC TGT ATA GGG-3', TNF-α forward primer 5'-AGG TTC TGT CCC TTT CAC TCA CTG -3 ', TNF-α reverse primer 5'-AGA GAA CCT GGG AGT CAA GGT A-3', IL-12 forward primer 5'-AGA GGT GGA CTG GAC TCC CGA -3', IL-12 reverse primer 5'-TTT GGT GCT TCA CAC TTC AG-3', β-actin forward primer 5'-TGG AAT CCT GTG GCA TCC ATG AAA C -3', β-actin reverse primer 5'-TAA AAC GCA GCT CAG TAACAG TCC G-3 '.

### Time-lapse imaging

By using Culture-insert m-dish^{35mm} (ibidi GmbH, Martinsried, Germany) for image capturing to confirm cell migration, MSCs (70 mL, 0.3 × 10⁶ cells/mL) were seeded on the left side, and B cells (70 mL, 3 × 10⁶ cells/mL) were seeded on the right side. After the cells were stabilized, inserts were removed and photographed using Biostation IM-Q (Nikon, Tokyo, Japan). In this experiment, photographing was performed for 3 hours to 6 hours at 2-minute intervals, and data analysis was performed by using the Imaris 7.2 software (Bitplane Inc., South Windsor, CT, USA).

### Chemotaxis assay

For the migration of B cells, 24-transwell plates (Costar, Corning, NY, USA) with a 5 µm insert upper well were used. B cells (1 × 10⁵ cells/well, upper chamber) isolated from PBMC and I3A-treated hMSCs (1 × 10³ cells/well, lower chamber) were co-cultured with ODN for 72 hours using a transwell. The level of IgM in the supernatant was measured by ELISA.

### Statistical analysis

Statistical analysis of experimental data was performed using the GraphPad Prism 5.0 (GraphPad, San Diego, CA, USA) software.

### Example. 1 Analysis of the effect of PMA-treated MSC on B cell function

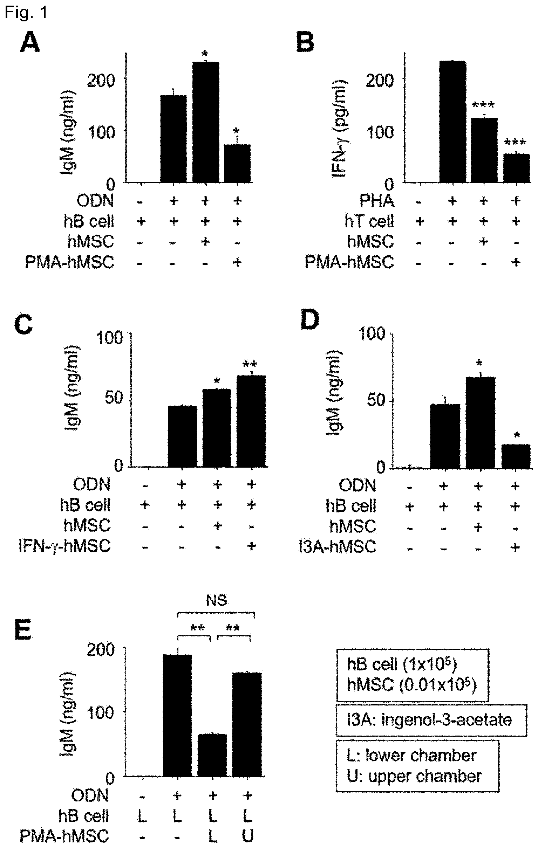

After B cells (1 × 10⁵ cells/well) isolated from peripheral blood mononuclear cells (PBMC) and PMA-treated hMSC (1 × 10³ cells/well) were co-cultured for 72 hours with CpG oligodeoxynucleotides (ODN, 5 µg/mL), the level of IgM in the supernatant was measured by ELISA (A in FIG. 1). T cells (1 × 10⁵ cells/well) isolated from PBMC and PMA-treated hMSCs (1 × 10³ cells/well) were co-cultured for 72 hours with phytohemagglutinin (PHA, 5 ug/mL), and the level of IFN-γ in the supernatant was measured by ELISA (B in FIG. 1). After B cells isolated from PBMC (1 × 10⁵ cells/well) and IFN-γ-treated hMSC (1 × 10³ cells/well) were co-cultured with ODN for 72 hours, the level of IgM in the supernatant was measured by ELISA (C in FIG. 1). B cells (1 × 10⁵ cells/well) isolated from PBMC and I3A-treated hMSC (1 × 10³ cells/well) were co-cultured with ODN for 72 hours, and the level of IgM in the supernatant was measured by ELISA (D in FIG. 1). B cells isolated from PBMC (1 × 10⁵ cells/well) and PMA-treated hMSC (1 × 10³ cells/well) were co-cultured with ODN for 72 hours using a transwell, and the level of IgM in the supernatant was measured by ELISA (E in FIG. 1).

Whereas naive MSC did not inhibit IgM production in B cells, PMA-treated MSC inhibited IgM production in B cells (A in FIG. 1). MSC inhibited IFN-γ production of T cells by about 50%, and PMA-treated MSC inhibited IFN-γ production of T cells by 77% (B in FIG. 1). Since it has been reported that IFN-γ enhances the immunosuppressive capacity of MSC, IFN-γ at 10 ng/mL was treated for 7 days, and the experiment was conducted. As a result of the experiment, it was confirmed that IFN-γ-treated MSC rather increased the IgM production of B cells (C in FIG. 1). I3A which is another PKC activator (ingenol 3-angelate, 10 ug/mL) was treated for 24 hours to conduct the experiment. It was confirmed that I3A-treated MSC inhibited IgM production of B cells (D in FIG. 1). Also, in order to find out by which method PMA-treated MSC inhibited IgM of B cells among cell-cell contact and soluble factors, it was analyzed using a transwell. Whereas PMA-treated MSC inhibited the IgM production of B cells, it was confirmed that it did not inhibit the IgM production of B cells, when contact between B cells and PMA-treated MSC was prevented using a transwell (E in FIG. 1). That is, whereas MSC induces IgM production of B cells, it was confirmed that PMA-treated MSC inhibits IgM production of B cells in a cell-cell contact dependent manner.

### Example 2. Analysis of the effect of PMA-treated MSC on B cell migration

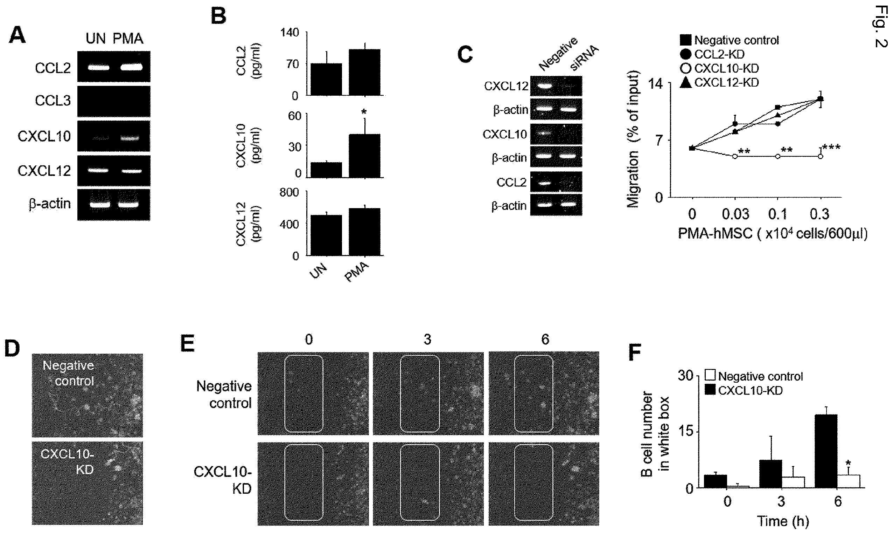

The chemokines of MSC were analyzed by RT-PCR (A in FIG. 2) and ELISA (B in FIG. 2) at mRNA and protein levels, respectively. After knocking down (KD) CCL2, CXCL10, and CXCL12 of MSC using siRNA, the mRNA level was confirmed via RT-PCR. The migration of B cells with respect to MSC was measured using a transwell, MSCs were seeded in the lower well, and B cells were seeded in the upper well. After 1.5 hours, the number of cells was counted using the fluorescence activated cell sorter (FACS) (C in FIG. 2). By using Culture-insert m-Dish^{35mm}, MSCs (70 mL of 0.3 × 10⁶ cells/mL) were added on the left side, and B cells (70 mL of 3 × 10⁶ cells/mL) were added on the right side. Then, it was filmed for 6 hours using time-lapse imaging, and the movement of representative B cells was marked with a green track (D in FIG. 2). The migration of B cells was observed by taking snapshots of the captured movies by each time period (E in FIG. 2). The number of T cells in the white box of the snapshot was shown as a graph (F in FIG. 2).

As PMA-treated MSCs inhibited IgM of B cells by cell-cell contact, migration and contact dynamics between the two cells were studied, and research was primarily conducted on chemokine signaling which is important for the migration of cells. It was confirmed that MSC expressed CCL2 and CXCL12, and when treated with PMA, the expression of CXCL10 increased (A and B in FIG. 2). When the expression of CXCL10 secreted from PMA-treated MSC was knocked down (KD), it was confirmed that B cells could not migrate toward the PMA-treated MSC (C in FIG. 2). In order to confirm this once again, image capturing was conducted. Whereas B cells migrated toward the control group PMA-treated MSC, it was confirmed that B cells did not migrate toward the PMA-treated MSC in which CXCL10 was knocked down (D and E in FIG. 2). As a result of counting the number of B cells in the box of the captured image, it was confirmed that the number of B cells which were co-cultured with PMA-treated CXCL10-KD MSC (PMA-treated and CXCL10-KD MSC) was less than that of the control group (F in FIG. 2).

### Example 3. Analysis of the effect of PMA-treated MSC on B cell contact

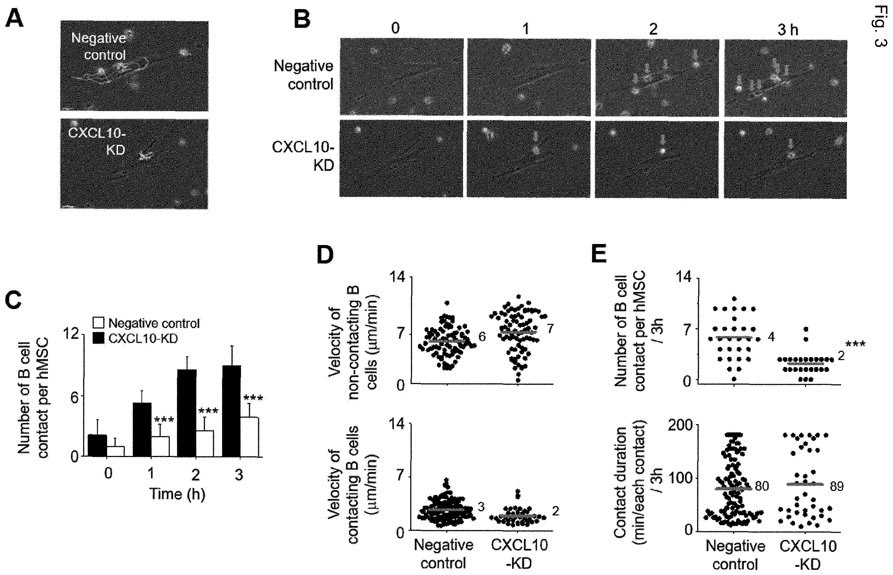

By using Culture-Dish^{35 mm}, MSCs (0.2 × 10⁶ cells/mL) and B cells (2 × 10⁶ cells/mL) were added and photographed for 3 hours using time-lapse imaging, and then, the movement of representative B cells was marked with a green track (A in FIG. 3). Snapshots of filmed movies were taken for each time period, and the contact between the B cells and MSCs was marked with a green arrow (B in FIG. 3). The number of B cells contacting the MSC was analyzed by each time period (C in FIG. 3). The rate of B cells not in contact with MSC and the rate of B cells in contact with MSC were displayed in a graph (D in FIG. 3). The number of B cells contacting one MSC and the contact time when the MSC and B cells were in contact were analyzed (E in FIG. 3).

In order to confirm the contact between the PMA-treated MSCs and B cells at a single cell level, the two cells were directly mixed and analyzed. After mixing the PMA-treated MSCs with the B cells, imaging was performed for 6 hours at 2-minute intervals, and it was confirmed that compared to the control group PMA-treated MSCs, the contact of B cells with respect to PMA-treated MSCs in which CXCL10 was knock down (KD) was reduced (A in FIG. 3). Even when viewed by each time period, compared to the control group PMA-treated MSCs, the contact of B cells with PMA-treated MSCs in which CXCL10 was knocked down was not increased (B and C in FIG. 3). Regardless of the type of PMA-treated MSCs, B cells which did not contact the PMA-treated MSCs showed a rate of about 6 µm/min to 7 µm/min, and B cells that contacted showed a rate of about 2 µm/min to 3 µm/min (D in FIG. 3). As a control group, the PMA-treated MSCs were in contact with B cells 4 times on average over 3 hours, and each contact was made for 80 minutes. On the other hand, PMA-treated MSCs in which CXCL10 was knocked down (CXCL10-KD PMA-treated MSC) were in contact with B cells twice on average for 3 hours, and it was confirmed that it took 89 minutes for each contact (E in FIG. 3). That is, it was confirmed that CXCL10 secreted from PMA-treated MSC regulates contact with B cells.

### Example 4. Analysis of the effect of PD-L1 expressed in PMA-treated MSC on B cell function

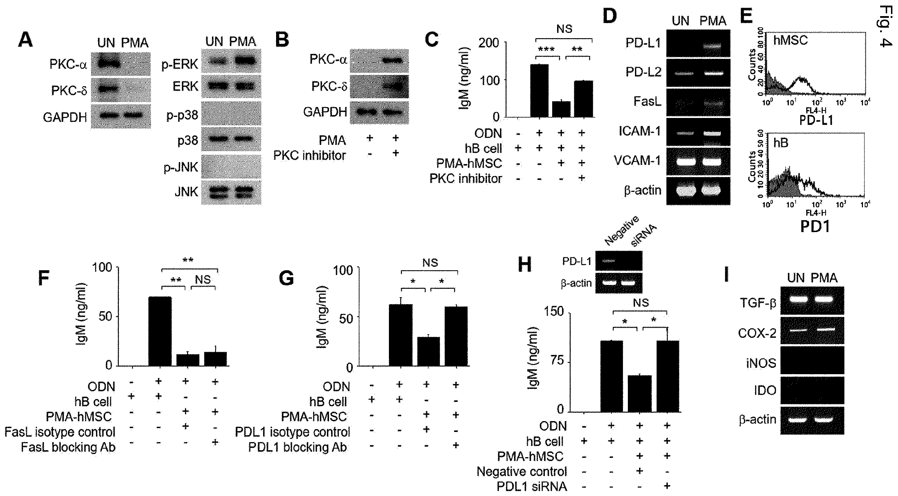

PMA was treated to MSC, and after 24 hours, proteins were obtained, and signaling was analyzed through western blot (A in FIG. 4). A PKC inhibitor (Go6983, 1 ug/mL) was pre-treated to MSC for 1 hour, and after PMA was treated, proteins were obtained after 24 hours, and signaling was analyzed through western blot (B in FIG. 4). After MSC was pretreated with the PKC inhibitor (Go6983, 1 ug/mL) for 1 hour and PMA was treated, B cells (1 × 10⁵ cells/well) that were isolated from PBMC and PMA-treated MSCs (1 × 10³ cells/well) were co-cultured with ODN for 72 hours. Then, the level of IgM in the supernatant was measured by ELISA (C in FIG. 4). The mRNA levels of the surface molecules of the PMA-treated MSCs were analyzed via RT-PCR (D in FIG. 4). The expressions of PD-L1 of the PMA-treated MSC and PD1 of the B cells were analyzed through FACS (E in FIG. 4). After treating the FasL blocking Ab (10 ug/mL) (F) and the PD-L1 blocking Ab (10 ug/mL) (G) to MSCs and adding PMA, MSCs and B cells were cultured with ODN for 72 hours, and then, the IgM level in the supernatant was measured by ELISA. The PMA-treated MSCs were treated with siRNA to confirm PD-L1 at the mRNA level via RT-PCR. After the PMA-treated MSCs, in which PD-L1 was knocked down, and B cells were cultured with ODN for 72 hours, the IgM level in the supernatant was measured by ELISA (H in FIG. 4). PMA was treated to the MSCs, and after 24 hours, the total RNA was obtained, and soluble factors were analyzed via RT-PCR (I in FIG. 4).

PMA is a PKC activator that activates PKC in the cytoplasm and moves it to the plasma membrane. When PMA was treated to MSC, it was confirmed that PKC-α and δ in the cytoplasm were transferred to the cell membrane, and phosphorylation of ERK was increased (A in FIG. 4). When the PKC inhibitor was pretreated to MSC, it was confirmed that the cell membrane migration of PKC-α and δ was inhibited due to PMA (B in FIG. 4), and it was confirmed that the IgM production of B cells was not inhibited (C in FIG. 4). When PMA was treated to MSC, it was confirmed that PD-L1, PD-L2, and FasL were expressed (D and E in FIG. 4). When the FasL blocking Ab was treated to PMA-treated MSCs, IgM production of B cells was inhibited (F in FIG. 4), but when the PD-L1 blocking Ab was treated, IgM production of B cells was not inhibited (G in FIG. 4). In addition, when PD-L1 expression was knocked down (KD) in the PMA-treated MSCs, IgM production of B cells was not inhibited (H in FIG. 4). When PMA was treated to MSC, intracellular TGF-β, COX-2, iNOS, and IDO expressions were not affected (I in FIG. 4). That is, it was confirmed that PMA suppresses IgM production of B cells by inducing PD-L1 expression of MSC.

### Example 5. Analysis of the effect of PDL1 expressed in PMA-treated MSC on B cell apoptosis

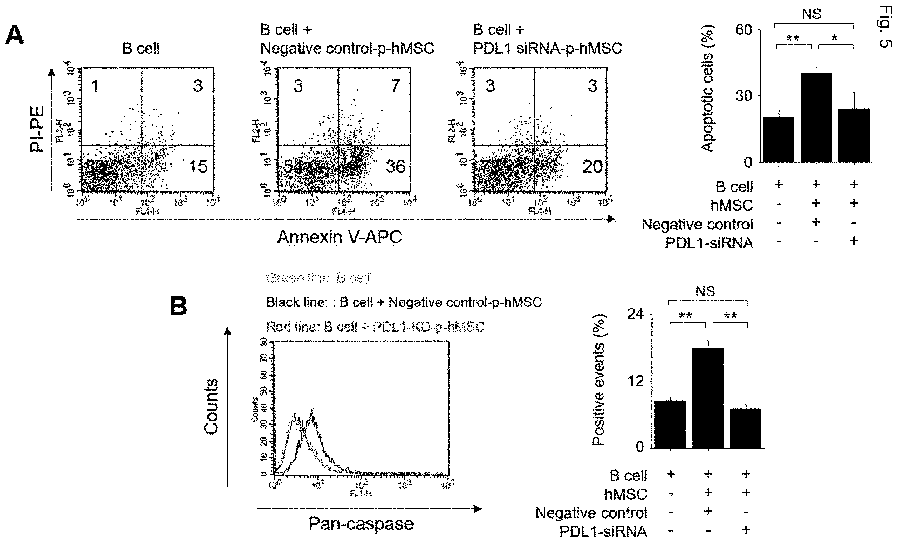

After PDL1 siRNA was treated to PMA-treated MSC, it was co-cultured for 24 hours, and the cells were stained using annexin V-APC and PI-PE and analyzed by FACS (A in FIG. 5). After PDL1 siRNA was treated to PMA-treated MSC, it was co-cultured for 24 hours, and then, caspase Ab was added, and it was analyzed by FACS after 1 hour (B in FIG. 5).

PD-L1 induces the death of cells expressing PD1. In this experiment, it was verified whether PD-L1 of the PMA-treated MSC induces B cell death by binding to PD1 on the B cell surface. When PD-L1 binds to PD1, caspase in the B cells is activated, and apoptosis is induced. When apoptosis proceeds, phosphatidic serine (PS) inside the cell membrane is exposed to the outside of the cell membrane, and as annexin V binds thereto, apoptosis can be measured. PMA-treated MSC strongly increased the expression of caspase in B cells (B in FIG. 5) and increased annexin V staining (A in FIG. 5). However, PD-L1 siRNA-transfected MSC did not increase caspase activation and annexin V staining of B cells (A and B in FIG. 5). From the above results, it can be known that PMA increases the expression of PD-L1 of MSC and increases B cell apoptosis by binding to PD1 of B cells.

### Example 6. Confirmation of therapeutic effect for MRL/lpr mice by administration of PMA-treated hMSC

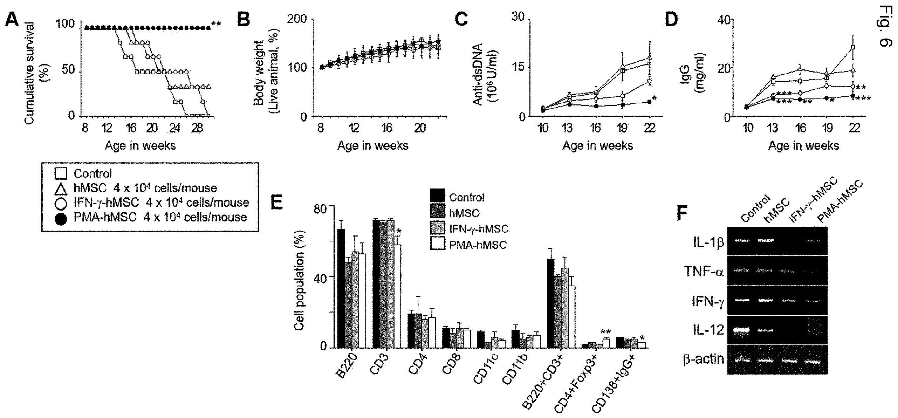

In order to proceed with the therapeutic effect experiment on PMA-treated MSC, the therapeutic effect was verified through MRL/lpr mice, which are a naturally occurring lupus animal model. MSC was injected intravenously into the mice at a concentration of 4 × 10⁴ cells/mouse, and the time of administration was injected once from 12 weeks of age when the onset of disease began in the mice (the control group (diluted solution) and MSC 4 × 10⁴ cells/injection were administered at 12 weeks of age of the MRL/lpr mice,). As a measurement index of animal experiments, the survival rate and body weight of the mice were checked every week, and anti-dsDNA and IgG were measured every two weeks. Survival rate (A in FIG. 6) and body weight (B in FIG. 6) were measured weekly, and serum was separated at 3-week intervals to measure the anti-dsDNA Ab (C in FIG. 6) and total IgG (D in FIG. 6). Cells were obtained by separating spleens from the MRL/lpr mice at 24 weeks of age, the cells were stained using antibodies, and the cell phenotype was measured by FACS (E in FIG. 6). Then, after obtaining the total RNA of the cells, the expression of inflammatory cytokines was measured via RT-PCR (F in FIG. 6).

The mice in the PMA-treated MSC administration group survived 100% until the age of 30 weeks, but the mice in the IFN-γ-treated MSC and MSC administration groups survived only 0% and 33%, respectively (A in FIG. 6). MSC did not affect the body weight of the MRL/lpr mice, from which it can be known that MSC does not show toxicity in mice (B in FIG. 6). Whereas PMA-treated MSC and IFN-γ-treated MSC inhibited the concentrations of the anti-dsDNA Ab and total IgG in the blood compared to the control group, MSC did not suppress the concentration of the anti-dsDNA Ab and total IgG (C and D in FIG. 6). The PMA-treated MSC decreased the ratio of CD3, which is a T cell phenotype, and the ratio of CD138+IgG+, which is a plasma cell phenotype, compared to the control group, and it increased the ratio of CD4+Foxp3+, which is a Treg cell phenotype (E in FIG. 6). In addition, as a result of analyzing the cytokine expression level viaRT-PCR, it was confirmed that the PMA-treated MSC reduces the expression level of inflammatory cytokines compared to the control group (F in FIG. 6).

### Example 7. Immune cell analysis in kidneys of MRL/lpr mice by administration of PMA-treated hMSC

After administering the control group (diluted solution) and MSC (4 × 10⁴ cells/injection) to MRL/lpr mice at 12 weeks of age, kidneys were separated from 24-week-old MRL/lpr mice and fixed with formalin, and the degree of infiltration of immune cells was measured using DAB staining.

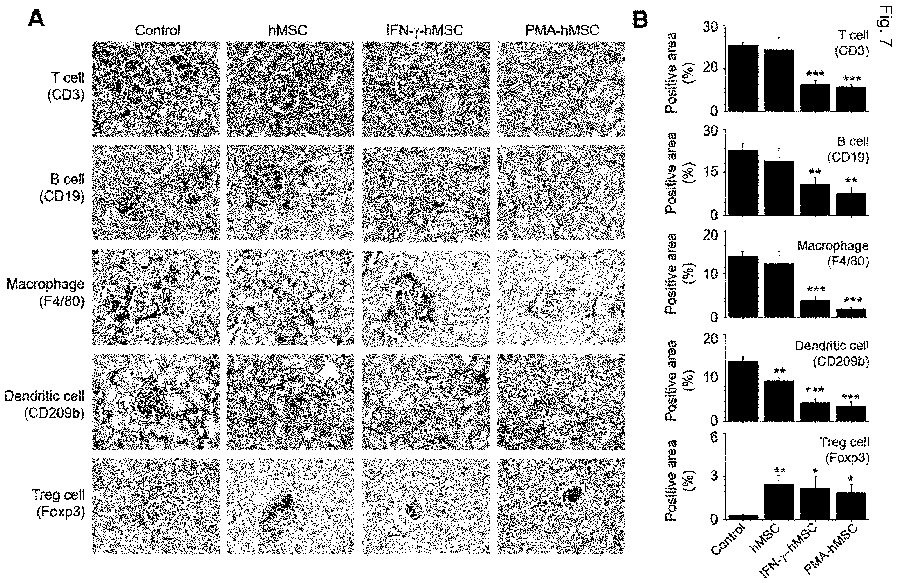

In the lupus disease, inflammation occurs at the kidneys by autoantibodies and immune complexes, and the infiltration of immune cells increases. Thus, the kidneys of the mice were separated, and the degree of cell infiltration was confirmed through DAB staining. PMA-treated MSC reduced kidney infiltration of T cells, B cells, macrophages, and dendritic cells compared to the control group, and increased the infiltration of Treg (A and B in FIG. 7). In conclusion, the PMA-treated MSC showed a therapeutic effect for the lupus disease, and showed a superior treatment effect compared to the existing MSC.

The above description of the present invention is for illustration only, and those skilled in the art to which the present invention pertains can understand that the present invention can be easily modified into other specific forms without changing the technical spirit or essential features of the present invention. Therefore, it should be understood that the exemplary embodiments described above are illustrative in all respects and not restrictive.

## Claims

1. A pharmaceutical composition for preventing or treating an autoimmune disease, comprising stem cells treated with a protein kinase C activator or a culture thereof.

2. The pharmaceutical composition of claim 1, wherein the protein kinase C activator comprises one or more selected from the group consisting of phorbol myristate acetate, ingenol 3-angelate, bryostatin-1, phorbol-12,13-dibutyrate, prostatin, N-(6-phenylhexyl)-5-chloro-1-naphthalenesulfonamide, and 5-chloro-N-heptylnaphthalene-1-sulfonamide.

3. The pharmaceutical composition of claim 1, wherein the stem cells are adult stem cells, pluripotent stem cells, induced pluripotent stem cells, or embryonic stem cells.

4. The pharmaceutical composition of claim 1, wherein the adult stem cells are mesenchymal stem cells, mesenchymal stromal cells, or multipotent stem cells.

5. The pharmaceutical composition of claim 1, wherein the protein kinase C activator increases the expression of CXCL10 in stem cells.

6. The pharmaceutical composition of claim 1, wherein the protein kinase C activator increases apoptosis of B cells by increasing the expression of programmed death-ligand 1 (PD-L1) in stem cells.

7. The pharmaceutical composition of claim 1, wherein the autoimmune disease is selected from the group consisting of lupus (systemic lupus erythematosus), rheumatoid arthritis, progressive systemic sclerosis (scleroderma), atopic dermatitis, alopecia areata, psoriasis, pemphigus, asthma, aphthous stomatitis, chronic thyroiditis, inflammatory enteritis, Behcet's disease, Crohn's disease, dermatomyositis, polymyositis, multiple sclerosis, autoimmune hemolytic anemia, autoimmune encephalomyelitis, myasthenia gravis, Grave's disease, polyarteritis nodosa, ankylosing spondylitis, fibromyalgia syndrome, and temporal arteritis.

8. A method for preventing or treating an autoimmune disease, comprising administering the pharmaceutical composition of claim 1 to a subject other than humans.

9. A method for preparing an immunosuppressive agent, comprising culturing by adding a protein kinase C activator to stem cells.

10. A method for inhibiting an immune response of a subject other than humans, comprising administering stem cells treated with a protein kinase C activator or a culture thereof to the subject.

11. A method for preventing or treating an autoimmune disease, comprising administering or feeding a pharmaceutical composition comprising stem cells treated with a protein kinase C activator or a culture thereof to a subject.

12. Use of a pharmaceutical composition comprising stem cells treated with a protein kinase C activator or a culture thereof in the prevention or treatment of an autoimmune disease.


<110>     CoreStem CO.,LTD.

<120>     PHARMACEUTICAL COMPOSITION COMPRISING STEM CELLS TREATED WITH
          PROTEIN KINASE C ACTIVATOR OR CULTURE THEREOF FOR PREVENTION AND
          TREATMENT OF AUTOIMMUNE DISEASES

<130>     PCT5040319

<150>     KR 10-2018-0137578
<151>     2018-11-09

<160>     36

<170>     KoPatentIn 3.0

<210>     1
<211>     25
<212>     DNA
<213>     Artificial Sequence

<220>
<223>     CCL2_F primer


<400>     1
atgaaagtct ctgccgccct tctgt                                              25


<210>     2
<211>     25
<212>     DNA
<213>     Artificial Sequence

<220>
<223>     CCL2_R primer


<400>     2
agtcttcgga gtttgggttt gcttg                                              25


<210>     3
<211>     24
<212>     DNA
<213>     Artificial Sequence

<220>
<223>     CCL3_F primer


<400>     3
atgcaggtct ccactgctgc cctt                                               24


<210>     4
<211>     25
<212>     DNA
<213>     Artificial Sequence

<220>
<223>     CCL3_R primer

<400>    4
gcactcagct ccaggtcgct gacat                                          25


<210>    5
<211>    20
<212>    DNA
<213>    Artificial Sequence

<220>
<223>    CXCL10_F primer


<400>    5
cctgcttcaa atatttccct                                                20


<210>    6
<211>    20
<212>    DNA
<213>    Artificial Sequence

<220>
<223>    CXCL10_R primer


<400>    6
ccttcctgta tgtgtttgga                                                20


<210>    7
<211>    22
<212>    DNA
<213>    Artificial Sequence

<220>
<223>    CXCL12_F primer


<400>    7
atgaacgcca aggtcgtggt cg                                             22


<210>    8
<211>    19
<212>    DNA
<213>    Artificial Sequence

<220>
<223>    CXCL12_R primer


<400>    8
tgttgttgtt cttcagccg                                                 19


<210>    9
<211>    20
<212>    DNA
<213>    Artificial Sequence

<220>
<223>    COX-2_F primer

<400>    9
tccttgctgt tcccacccat                                                    20


<210>    10
<211>    20
<212>    DNA
<213>    Artificial Sequence

<220>
<223>    COX-2_R primer


<400>    10
catcatcaga ccaggcacca                                                    20


<210>    11
<211>    20
<212>    DNA
<213>    Artificial Sequence

<220>
<223>    iNOS_F primer


<400>    11
acgtgcgtta ctccaccaac                                                    20


<210>    12
<211>    20
<212>    DNA
<213>    Artificial Sequence

<220>
<223>    iNOS_R primer


<400>    12
catagcggat gagctgagca                                                    20


<210>    13
<211>    21
<212>    DNA
<213>    Artificial Sequence

<220>
<223>    IDO_F primer


<400>    13
agcctgatct catagagtct g                                                  21


<210>    14
<211>    20
<212>    DNA
<213>    Artificial Sequence

<220>
<223>    IDO_R primer


<400>    14
ttactgcagt ctccatcacg                                                    20


<210>    15
<211>    18
<212>    DNA
<213>    Artificial Sequence

<220>
<223>    TGF-beta_F primer


<400>    15
cagatcctgt ccaagctg                                                      18


<210>    16
<211>    18
<212>    DNA
<213>    Artificial Sequence

<220>
<223>    TGF-beta_R primer


<400>    16
tcggagctct gatgtgtt                                                      18


<210>    17
<211>    23
<212>    DNA
<213>    Artificial Sequence

<220>
<223>    FasL_F primer


<400>    17
ggattgggcc tggggatgtt tca                                                23


<210>    18
<211>    24
<212>    DNA
<213>    Artificial Sequence

<220>
<223>    FasL_R primer


<400>    18
ttgtggctca ggggcaggtt gttg                                               24


<210>    19
<211>    20
<212>    DNA

<213>    Artificial Sequence

<220>
<223>    PD-L1_F primer


<400>    19
ttgggaaatg gaggataaga                                                    20


<210>    20
<211>    21
<212>    DNA
<213>    Artificial Sequence

<220>
<223>    PD-L1_R primer


<400>    20
ggatgtgcca gaggtagttc t                                                  21


<210>    21
<211>    16
<212>    DNA
<213>    Artificial Sequence

<220>
<223>    PD-L2_F primer


<400>    21
acaccgtgaa agagcc                                                        16


<210>    22
<211>    17
<212>    DNA
<213>    Artificial Sequence

<220>
<223>    PD-L2_R primer


<400>    22
aatgtgaagc agccaag                                                       17


<210>    23
<211>    20
<212>    DNA
<213>    Artificial Sequence

<220>
<223>    ICAM1_F primer


<400>    23
cgtgccgcac tgaactggac                                                    20


<210>    24

<211>    21
<212>    DNA
<213>    Artificial Sequence

<220>
<223>    ICAM1_R primer


<400>    24
cctcacactt cactgtcacc t                                                21


<210>    25
<211>    19
<212>    DNA
<213>    Artificial Sequence

<220>
<223>    VCAM1_F primer


<400>    25
atgacatgct tgagccagg                                                   19


<210>    26
<211>    20
<212>    DNA
<213>    Artificial Sequence

<220>
<223>    VCAM1_R primer


<400>    26
gtgtctcctt ctttgacact                                                  20


<210>    27
<211>    20
<212>    DNA
<213>    Artificial Sequence

<220>
<223>    beta-actin_F primer


<400>    27
gtggggcgcc ccaggcacca                                                  20


<210>    28
<211>    20
<212>    DNA
<213>    Artificial Sequence

<220>
<223>    beta-actin_R primer


<400>    28
ctccttaatg tcacgcacga                                                  20

<210>    29
<211>    26
<212>    DNA
<213>    Artificial Sequence

<220>
<223>    INF-gamma_F primer


<400>    29
agcggctgac tgaactcaga ttgtag                                          26


<210>    30
<211>    24
<212>    DNA
<213>    Artificial Sequence

<220>
<223>    INF-gamma_R primer


<400>    30
gtcacagttt tcagctgtat aggg                                            24


<210>    31
<211>    24
<212>    DNA
<213>    Artificial Sequence

<220>
<223>    INF-alpha_F primer


<400>    31
aggttctgtc cctttcactc actg                                            24


<210>    32
<211>    22
<212>    DNA
<213>    Artificial Sequence

<220>
<223>    INF-alpha_R primer


<400>    32
agagaacctg ggagtcaagg ta                                              22


<210>    33
<211>    21
<212>    DNA
<213>    Artificial Sequence

<220>
<223>    IL-12_F primer


<400>    33

agaggtggac tggactcccg a                                                21

<210>    34
<211>    19
<212>    DNA
<213>    Artificial Sequence

<220>
<223>    IL-12_R primer

<400>    34
ttggtgcttc acacttcag                                                  19

<210>    35
<211>    25
<212>    DNA
<213>    Artificial Sequence

<220>
<223>    beta-actin_F primer

<400>    35
tggaatcctg tggcatccat gaaac                                           25

<210>    36
<211>    25
<212>    DNA
<213>    Artificial Sequence

<220>
<223>    beta-actin_R primer

<400>    36
taaaacgcag ctcagtaaca gtccg                                           25